# EUROPEAN PATENT APPLICATION

(11) **EP 1 757 574 A1**
(43) Date of publication of application: **28.02.2007**
(21) Application number: 05730643.3
(22) Date of filing: 15.04.2005
(51) Int. Cl.: C07C 67/11, C07C 69/653, C08F 20/22

(54) **PROCESS FOR PRODUCING FLUORINATED (METH)ACRYLIC ESTER**

(30) Priority: 26.04.2004 JP 2004129710
(71) Applicant: DAIKIN INDUSTRIES, LTD., Osaka-shi, Osaka 530-8323 (JP)
(72) Inventor: FUNAKOSHI, Yoshio, DAIKIN INDUSTRIES LTD., Settsu-shi, Osaka 5668585 (JP); TANAKA, Yoshinori, DAIKIN INDUSTRIES LTD., Settsu-shi, Osaka 5668585 (JP); HIRASAKA, Takeomi, DAIKIN INDUSTRIES LTD., Settsu-shi, Osaka 5668585 (JP)
(74) Representative: HOFFMANN EITLE
(86) International application number: PCT/JP2005/007324
(87) International publication number: WO 2005/102982

(57) **Abstract**

A mixture of fluoroalkyl iodides of the formula C₂F₅(CF₂CF₂)ₙI ("n" is an integer of ≥0) wherein the sum of such fluoroalkyl iodides of n=3 and n=4 contained is ≥85 mol% is provided. This mixture is subjected to an ethylene addition step and an esterification step, and C₂F₅(CF₂CF₂)ₙCH=CH₂ and C₂F₅(CF₂CF₂)ₙCH₂CH₂OH are removed. Thus, there can be obtained a mixture of fluorine-containing (meth)acrylic esters of the formula C₂F₅(CF₂CF₂)ₙCH₂CH₂OCOCR¹=CH₂ (wherein R¹ represents a hydrogen atom or a methyl group, and "n" is an integer of ≥0) wherein the content of impurities (namely, olefins of the formula C₂F₅(CF₂CF₂)ₙCH=CH₂ and alcohols of the formula C₂F₅(CF₂CF₂)ₙCH₂CH₂OH) is low.

## Description

### Technical Field

The present invention is related to a method for producing a mixture of fluorine-containing (meth)acrylic esters which includes less impurities.

### Background Art

A fluorine-containing (meth)acrylic ester represented by a general formula (1):

C₂F₅(CF₂CF₂)ₙCH₂CH₂OCOCR¹=CH₂ (1)

wherein R¹ is a hydrogen atom or a methyl group and "n" is an integer of at least zero is used as a monomer in production of a fluorine-containing acrylate-based polymer that may be an active component for a water- and oil-repellent. Various methods for producing this monomer have been proposed.

For example, Japanese Patent Kokoku Publication No. 539-18112(1964) and Japanese Patent Kokoku Publication No. S48-30611(1973) disclose a method for reacting a fluorine-containing alkyl halide with an alkali metal salt of a carboxylic acid in a specific solvent. Japanese Patent Kokoku Publication Nos. H4-16451(1992), H4-16452(1992) and S61-57813(1986) disclose a method for producing the fluorine-containing ester and mention that the resultant fluorine-containing ester may be converted to an acrylic ester. Further, Japanese Patent Kokai (Laid-Open) Publication No. H9-59215(1997) discloses a method for producing the fluorine-containing (meth)acrylic ester represented by the above formula (1) is produced by reacting the fluorine-containing alkyl halide with a specific betaine followed by an alkaline treatment.

Another method for producing the fluorine-containing (meth)acrylic ester is known wherein the fluoroalkyl halide is converted to the fluorine-containing alkyl alcohol that is then converted to the fluorine-containing (meth)acrylic ester. For example, in Japanese Patent Kokai (Laid-Open) Publication No. S59-181239(1984), a method for producing the fluorine-containing (meth)acrylic ester is disclosed wherein the fluorine-containing alkyl alcohol and acrylic acid or methacrylic acid are reacted under the presence of a concentrated sulfuric acid or a fuming sulfuric acid. In Patent Kokai Publication (Laid-Open) No. H2-295948(1990), a method for reacting the fluorine-containing alkyl alcohol and methacrylic acid under the presence of phosphoric anhydride is disclosed. USP No. 3,719,698 discloses a method for producing the fluorine-containing (meth)acrylic ester wherein the fluorine-containing alkyl alcohol is added to a compound that is obtained by reacting acrylic acid or methacrylic acid with a trifluoroacetic anhydride. Japanese Patent Kokai (Laid-Open) Publication Nos. S59-117503(1984), S59-117504(1984) and H3-163044(1991) disclose a method for producing the fluorine-containing (meth)acrylic ester wherein the acrylic acid halide or the methacrylic acid halide is reacted with the fluorine-containing alcohol in an aqueous solution of an alkali metal hydroxide.

In any methods, a fluoroalkyl iodide generally represented by R_{f}I (R_{f} is a fluoroalkyl group) is used as a starting material. In the method disclosed in Patent Kokoku Publication Nos. S39-18112(1964) and S48-30611(1973), an ethylene adduct of this iodide is used. In the method using the fluoroalkyl alcohol as described in Japanese Patent Kokoku Publication No. S61-57813(1986), this iodide is used as a material for the alcohol. R_{f}I is, for example, a telomer produced by a telomerization reaction.

### Disclosure of Invention

### (Problems to be Solved by Invention)

It is known that when the monomers represented by the formula (1) are polymerized, the resultant product contains, in addition to the polymer, an olefin represented by the following formula (3) and an alcohol represented by the following formula (4):

C₂F₅(CF₂CF₂)ₙCH=CH₂ (3)

C₂F₅(CF₂CF₂)ₙCH₂CH₂OH (4)

These compounds do not function as the active component for the water- and oil-repellent.

Further, it is known that the preferable monomers constituting the polymer for the water- and oil-repellent are ones with "n" of at least 3 in the above formula (1). When the polymerization is carried out using a mixture of the monomers with "n" of at least 3, compounds represented by the formulas (3) and (4) of n=3, that is, C₈F₁₇CH=CH₂ and C₈F₁₇CH₂CH₂OH are contained in the polymer. Of these compounds, an alcohol with "n" of three, that is, C₈F₁₇CH₂CH₂OH may be oxidized to give C₇F₁₅COOH (perfluorooctanoic acid: abbreviation PFOA). Recent research results for example, EPA report "PRELIMINARY RISK ASSESSMENT OF THE DEVELOPMENTAL TOXICITY ASSOCIATED WITH EXPOSURE TO PERFLUOROOCTANOIC ACID AND ITS SALTS" (http://www.epa.gov/opptintr/pfoa/pfoara.pdf) point out that perfluorooctanoic acid might be a burden to the environment. For this reason, the EPA (Environmental Protection Agency of US) announced, on April 14, 2003, that they would strengthen the scientific research as to PFOA.

The compounds represented by the formulas (3) and (4) are preferably removed from the polymer from the viewpoint of providing a product of high quality as described above, and thereby it may be possible to avoid the inclusion of PFOA in the final product. One method for preventing these compounds from being contained in the polymer is a technique of removing these compounds in the monomer stage by rectification. However, the olefin and the alcohol are generally produced as mixtures of the compounds with "n" of at least zero, and these compounds may have, depending on "n", boiling points close to that of the fluorine-containing (meth) acrylic ester to be used for the polymerization. For this reason, it is difficult to remove all the impurities by a simple operation in the monomer phase.

The present invention is made in light of these circumstances, and the object of the present invention is to provide a method for producing the fluorine-containing (meth)acrylic esters, which method makes it possible to produce the polymer containing less impurities.

### (Means to Solve the Problem)

The inventors found that most of by-products can be removed by rectification when a mixture of the ethylene adducts containing the adducts of n=3 and n=4 in a large amount is supplied to an esterification step in a method for producing a mixture of fluorine-containing (meth)acrylic esters wherein the fluoroalkyl iodide is used as a starting material which is subjected to the ethylene addition step and the esterification step that involves the reaction of the ethylene adduct with (meth)acrylic acid compound, and thereby the object is achieved. Specifically, the inventors found that the object is achieved by i) drawing, from a mixture of fluoroalkyl iodides represented by C₂F₅(CF₂CF₂)ₙI, another mixture containing the iodides of n=3 and n=4 in a large amount before the ethylene addition step and then producing the fluorine-containing (meth)acrylic esters using the another mixture, or ii) carrying out the ethylene addition step to give a mixture of the ethylene adducts represented by C₂F₅(CF₂CF₂)ₙCH₂CH₂I; drawing another mixture containing the adduct of n=3 and n=4 in a large amount; and then subjecting the another mixture to the esterification step.

In other words, the present invention provides, as a fist production method, a method for producing a mixture of fluorine-containing (meth)acrylic esters each of which esters is represented by the formula (1):

C₂F₅(CF₂CF₂)ₙCH₂CH₂OCOCR¹=CH₂ (1)

wherein R¹ is a hydrogen atom or a methyl group and "n" is an integer of at least zero, which includes:
(A) a step of obtaining a mixture of fluoroalkyl iodides each of which is represented by C₂F₅(CF₂CF₂)ₙI wherein "n" is an integer of at least zero which mixture contains the fluoroalkyl iodides of n=3 and n=4 in an amount of 85 mol% or more in total;
(B) a step of ethylene addition wherein ethylene is added to the mixture of the fluoroalkyl iodides obtained in the step (A) to give a mixture of ethylene adducts each of which is represented by the formula (2):

   C₂F₅(CF₂CF₂)ₙCH₂CH₂I (2)

   wherein "n" is an integer of at least zero;
(C) an esterification step of reacting the mixture of the ethylene adducts obtained in the step (B) with a (meth)acrylic compound to give a mixture containing the fluorine-containing (meth)acrylic esters; and
(D) a step of reducing a proportion of compounds represented by the formula (3) and a proportion of compounds represented by the formula (4) contained in the mixture obtained in the step (C):

   C₂F₅(CF₂CF₂)ₙCH=CH₂ (3)

   wherein "n" is an integer of at least zero,

   C₂F₅(CF₂CF₂)ₙCH₂CH₂OH (4)

   wherein "n" is an integer of at least zero.

Herein, the "mixture of fluorine-containing (meth)acrylic esters" refers to a mixture containing two or more esters which have different "n" values in the formula (1). The fluorine-containing (meth)acrylic ester is generally obtained as such a mixture, but is can be obtained as a compound wherein "n" value is a particular single numeral. However, it seems unlikely that no other esters with other "n" values are contained in the compound when the compound with "n" of the particular single numeral is obtained. For this reason, the term "mixture" is used herein. Similarly, the term "mixture" as to a compound which includes, in its chemical formula, a letter such as "n" or the like corresponding to a polymerization degree is used herein in the sense that it includes a plurality of compounds with different "n" values. Further, in this specification, a symbol "≥" may be used to generically refer to compounds with "n" of "k" or more (or at least "k") and a symbol "≤" may be used to generically refer to compounds with "n" of "k" or less (or at most "k"). Furthermore, a mixture which does not contain a compound with "n" of zero (specifically, the fluorine-containing (meth)acrylic ester, the fluoroalkyl iodide or the ethylene adduct) is identified as a mixture with "n" of one or more and such a mixture can be employed or produced in the present invention.

As the fluoroalkyl iodides represented by C₂F₅(CF₂CF₂)ₙI, the compounds of n=3 and n=4 are mainly used in this production method. Therefore, the compounds with n=3 and n=4 in the above formulas (3) and (4), that is, C₈F₁₇CH=CH₂, C₈F₁₇CH₂CH₂OH, C₁₀F₂₁CH=CH₂ and C₁₀F₂₁CH₂CH₂OH are mainly produced as the by-products. These compounds have boiling points which are significantly lower than those of the fluorine-containing (meth)acrylic esters and therefore can be separated by rectification. Further, according to the production method of the present invention, compounds which are derived from the fluoroalkyl iodides with "n" of 5 or more (that is, n≥5) and represented by the formulas (3) and (4) are not produced or produced in a minute amount. This fact contributes to improvement in purity of the fluorine-containing (meth)acrylic esters produced according to the method of the present invention. Since the boiling points of the compounds of n≥5 (particularly, the alcohol represented by the formula (4) with n=5) are close to those of the fluorine-containing (meth)acrylic esters with n=3 and n=4, the compounds are difficult to be separated from the esters by rectification and they tend to finally remain as the impurities in the polymer. The production method of the present invention makes it possible to reduce the impurities contained in the polymer since causative compounds of the production of such compounds do not exist in the starting material.

In the production method of the present invention, the mixture of the fluoroalkyl iodides represented by C₂F₅(CF₂CF₂)ₙI is used as the starting material, which mixture contains the iodides of n=3 and n=4 in an amount of 85 mol% or more in total (the step (A)). When the proportion of the fluoroalkyl iodides of n=3 and n=4 is below 85 mol%, the fluoroalkyl iodides with small "n" values or large "n" values are contained in a large amount. The fluorine-containing (meth)acrylic esters obtained from the fluoroalkyl iodides with the small "n" values do not form the polymer having good properties as the water- and oil-repellant, and therefore it is undesirable that such iodides are contained in a large amount. As described above, the fluorine-containing (meth)acrylic esters obtained from the fluoroalkyl iodides with the large "n" values cause the production of the olefins and the alcohols which are difficult to be separated from the fluorine-containing (meth)acrylic esters, and therefore it is undesirable that such esters are contained in a large amount.

The starting material may be only the fluoroalkyl iodide of n=3 or n=4. However, since the compound represented by C₂F₅(CF₂CF₂)ₙI is usually obtained as a mixture of the compounds with different "n" values, complicated operations and equipment are required so as to obtain, from the mixture, another mixture wherein "n" is a particular single integral (for example, a mixture which contains mainly the iodide of n=3 (or n=4) and the iodides with other "n" values in only a small amount not to be separated), resulting in economic disadvantage. The production method of the present invention is industrially advantageous in that the elimination of or the reduction in the C₂F₅(CF₂CF₂)ₙI with "n" of two or less (that is, n≤2) and C₂F₅(CF₂CF₂)ₙI with n≥5 enables the useful fluorine-containing (meth)acrylic esters with less impurities to be obtained even if the fluoroalkyl iodide is used in a form of the mixture.

In the production method of the present invention, the step (D) corresponds to a step of removing the olefins and the alcohols which are to be impurities from the mixture obtained in the step (C), and this step corresponds to a step of obtaining a mixture wherein the purity of the esters that are the objective compound is higher. In the production method of the present invention, the step (D) is preferably a distillation step. As described above, since the boiling points of the fluorine-containing olefins represented by the formula (3) with n=3 and n=4 and the fluorine-containing alcohols represented by the formula (4) with n=3 and n=4 are lower than those of the fluorine-containing (meth)acrylic esters that are the object, the distillation is an efficient method for obtaining the mixture with the mixing proportions of these compounds reduced, and suitable for an industrial mass production.

The present invention also provides, as a second production method, a method for producing a mixture of the fluorine-containing (meth)acrylic esters with less impurities;
(A') a step of obtaining a mixture of fluoroalkyl iodides each of which is represented by C₂F₅(CF₂CF₂)ₙI wherein "n" is an integer of at least zero;
(B) a step of ethylene addition wherein ethylene is added to the mixture of the fluoroalkyl iodides obtained in the step (A') to give a mixture of ethylene adducts each of which is represented by the formula (2):

   C₂F₅(CF₂CF₂)ₙCH₂CH₂I (2)

   wherein "n" is an integer of at least zero;
(B') a step of obtaining, from the mixture of the ethylene adducts obtained in the step (B), another mixture of ethylene adducts which contains the adducts of n=3 and n=4 in an amount of 85 mol% or more in total;
(C) an esterification step of reacting the mixture of the ethylene adducts obtained in the step (B') with a (meth)acrylic compound to give a mixture containing the fluorine-containing (meth)acrylic esters; and
(D) a step of reducing a proportion of compounds represented by the formula (3) and a proportion of compounds represented by the formula (4) contained in the mixture obtained in the step (C):

   C₂F₅(CF₂CF₂)ₙCH=CH₂ (3)

   wherein "n" is an integer of at least zero,

   C₂F₅(CF₂CF₂)ₙCH₂CH₂OH (4)

   wherein "n" is an integer of at least zero. The difference between the first and the second production methods is only the time when the compounds of n≤2 and n≥5 are removed and the effects and so on of the second production method are the same as those of the first production method.

The present invention further provides a method for producing a polymer by polymerizing the fluorine-containing (meth)acrylic esters produced by the first or the second production method. Since the starting materials (monomers) with less impurities are used in the method for producing a polymer of the present invention, the resultant polymer also contains less impurities.

### (Effect of Invention)

The production method of the present invention makes it possible to obtain the mixture of the fluorine-containing (meth)acrylic esters which contains less impurities. The ester mixture with less impurities improves the quality of the final product (for example, the water- and oil-repellent) which is obtained by polymerizing the esters. Further, the production of PFOA resulting from the impurities can be reduced.

### Embodiments for Carrying Out the Invention

Each step is described below to explain the method of the present invention for producing the mixture of the fluorine-containing (meth) acrylic esters. Each step conducted in the first production method is described first.

In the step (A), there is produced the mixture of the fluoroalkyl iodides represented by C₂F₅(CF₂CF₂)ₙI in which the sum of the proportions of the fluoroalkyl iodides of n=3 and n=4 is high. The mixture of the fluoroalkyl iodides represented by C₂F₅(CF₂CF₂)ₙI may be produced employing a conventional method. Specifically, the fluoroalkyl iodides may be produced by, for example, a telomerization reaction wherein C₂F₅I is a telogen and tetrafluoroethylene is a taxogen. The method thereof is known, and therefore the details thereof are omitted herein.

The fluoroalkyl iodide is generally obtained as a mixture of compounds with different "n" values, as is the case with the telomer obtained by the telomerization reaction. The fluoroalkyl iodides with n≤2 and n≥5 need to be removed by conducting, for example, a distillation operation in order that the mixture becomes a mixture which contains the fluoroalkyl iodides of n=3 and n=4 at a proportion of 85 mol% or more. Specifically, the desired and intended mixture can be obtained by distilling off the fluoroalkyl iodides of n≤2 from, for example, the column top and withdrawing the fluoroalkyl iodides of n=3 and n=4 from the side of the rectification column by distillation. In that case, the fluoroalkyl iodides of n≥5 are withdrawn as bottom or still residue. Alternatively, the desired and intended mixture can be obtained by distilling off the fluoroalkyl iodides of n≤2 and then withdrawing the fluoroalkyl iodides of n=3 and n=4 from the column top. Alternatively, the desired and intended mixture may be obtained ay a method wherein a plurality of rectifiers are connected; the fluoroalkyl iodides with smaller "n" values are removed sequentially; and the fluoroalkyl iodides of n=3 and n=4 are withdrawn from the final rectifier by being distilled off.

The distillation of the mixture of the fluoroalkyl iodides is preferably carried out so that the mixture is obtained in which the mixing proportion of the sum of the fluoroalkyl iodides with n≤2 and n≥5 is less than 15 mol% in the intended mixture which is to be subjected to the step (B), and more preferably carried out so that the mixture wherein the proportion of the fluoroalkyl iodides with n≤2 is less than 10 mol% and the proportion of the fluoroalkyl iodides with n≥5 is less than 5 mol% in the intended mixture. It is preferable that the proportion of the fluoroalkyl iodides with n≥5 is as low as possible since the iodides give the compounds which are difficult to be separated from the ester mixture. The specific conditions for distillation (generally, rectification) are the bottom temperature of from 60°C to 140°C, the pressure in the column of from 0.5kPa to 60kPa and theoretical plate number of from 5 to 25. However, a slight amount of the fluoroalkyl iodides with n≤2 and n≥5 may exist in the mixture even if any distillation conditions are employed. It should be noted that the preferable proportions of the fluoroalkyl iodides with n≤2 and n≥5 in the mixture are defined in the above considering such case.

The step (B) is a step of adding ethylene to the mixture of the fluoroalkyl iodides obtained in the step (A) to give the ethylene adducts represented by the formula (2):

C₂F₅(CF₂CF₂)ₙCH₂CH₂I (2).

The step (B) may be carried out under the conditions which are conventionally employed in the ethylene addition reaction. Specifically, the ethylene addition is carried out under at a reaction temperature is 30°C to 250°C, for example, 50°C to 220°C and a reaction pressure is 1MPa or less, for example, 0.2MPa to 0.4MPa. The reaction time is generally 0.1 hours to 10 hours. A reaction pressure is a pressure generated by the injected ethylene. The reaction is preferably carried out at a molar ratio of the mixture of fluoroalkyl iodides to ethylene of 1:2 to 1:1.05.

The ethylene addition reaction may be carried out under the presence of a catalyst which generates a radical. The catalysts include, for example, an azo compound, an organic peroxide, a metallic catalyst and a metallic salt catalyst. The azo compound suitable for the catalyst is, for example, for example α,α'-azobisisobutyronitrile. The organic peroxide suitable for the catalyst is a diacyl peroxide such as benzoyl peroxide, a dialkyl peroxide such as t-butyl peroxide, or a peroxymonocarbonate such as t-butyl peroxyisopropyl monocarbonate. The metallic catalyst suitable for the catalyst is, for example, copper, chromium, manganese, nickel, or platinum. The metallic salt catalyst suitable for the catalyst is, for example, a chloride of any of the aforementioned metals. The amount of the catalyst is from about 0.005 mol to about 0.02 mol per mole of the fluoroalkyl iodide mixture when the azo compound or the organic peroxide is used. The amount of the catalyst is from about 0.01 mol to about 0.1 mol per mole of the fluoroalkyl iodide mixture when the metallic catalyst or the metallic salt catalyst is used.

As a result of the step (B), the mixture of the ethylene adducts represented by the formula (2) is obtained. The proportion of each of the ethylene adducts with different "n" values in the ethylene adduct mixture is the same as that of each of the fluoroalkyl iodides with different "n" values in the fluoroalkyl iodide mixture employed. Therefore, the ethylene adducts of n=3 and n=4 occupy 85 mol% or more in total in the mixture obtained in the step (B).

The step (C) is a step of reacting the mixture of the ethylene adducts obtained in the step (B) with the (meth)acrylic acid compound to obtain the intended mixture of the fluorine-containing (meth)acrylic esters. The (meth)acrylic acid compound may be, for example, a metallic salt of (meth)acrylic acid. The metallic salts of (meth)acrylic acid include, for example, an alkaline metal (such as potassium or sodium) salt, or an alkaline earth metal salt. The step (C) may be carried out under the conditions which are conventionally employed in an esterification reaction. Specifically the step is carried out at a reaction temperature of from 160°C to 220°C, for example, 170°C to 190°C. The reaction time is generally 0.1 hours to 10 hours.

As described above, not only the esters of the formula (1), but also the olefins of the formula (3) and the alcohols of the formula (4) are produced as the by-products in the step (C). In the step (D), an operation is carried out to obtain a mixture wherein the proportion of these by-products is smaller. Such an operation is distillation, as described. The distillation is usually carried out as the rectification when it is conducted industrially. The preferable distillation method employed in the production method of the present invention is described below.

It is preferable that the distillation is conducted so that the mixing ratio of the sum of the fluorine-containing olefins represented by the formula (3) with "n" of 0, 1 and 2 is substantially 0 mol%, the mixing ratio of the fluorine-containing olefin with "n" of 3 is substantially 0 mol%, the mixing ratio of fluorine-containing olefin with "n" of 4 is substantially 0 mol%, and the mixing ratio of the sum of the fluorine-containing olefins with "n" of 5 or more is from 0 mol% to 0.1 mol%, all the ratios being ones to the total moles of the fluorine-containing compounds obtained as the distillate. Further, it is preferable that the distillation is conducted so that the mixing ratio of the sum of the fluorine-containing alcohols represented by the formula (4) with "n" of 0, 1 and 2 is substantially 0 mol%, the mixing ratio of the fluorine-containing alcohol with "n" of 3 is substantially 0 mol%, the mixing ratio of fluorine-containing alcohol with "n" of 4 is from 0 mol% to 0.05 mol%, and the ratio of the sum of the fluorine-containing alcohols with "n" of 5 or more is from 0 mol% to 0.1 mol%, all the ratios being ones to the total moles of the fluorine-containing compounds obtained as the distillate. In other words, the distillation is preferably carried out such that the proportions of the fluorine-containing olefins and the fluorine-containing alcohol which occupy the mixture of the fluorine-containing (meth)acrylic esters are reduced. Herein, it should be noted that the expression that a component is "substantially 0 mol%" means that the component cannot be detected by usual gas chromatography and the mixture may contain the component in a minute amount within that range. Specifically, such distillation is carried out at the bottom temperature of from 60°C to 160°C and the pressure in the column of from 0.5kPa to 5kPa with the theoretical plate number of from 10 to 35.

It is preferable that the distillation is carried out adding an inhibitor of polymerization to the mixture obtained in the step (C) in order to prevent the fluorine-containing (meth)acrylic esters from being polymerized in the distillation column. For example, hydroquinone or hydroquinone monomethyl ether may be added as the inhibitor of polymerization. When these inhibitors are used, it is preferable that the distillation is carried out introducing oxygen or a gas containing oxygen (for example, air) into the distillation column. The introduction of oxygen further suppresses the polymerization of the fluorine-containing (meth)acrylic esters. The inhibitor of polymerization used in the step (C) is not necessarily required to be hydroquinone or hydroquinone monomethyl ether and may be another one.

Further, a resin lining is preferably used in the distillation column. Furthermore, a resin packing is preferably packed in the distillation column. Metal is excluded from the interior of the distillation column by forming the members inside the distillation column of a resin as described above. As a result, the deterioration of the inhibitor of polymerization is effectively prevented during the distillation, and therefore the polymerization of the fluorine-containing (meth)acrylic esters can be more effectively prevented during the distillation. Alternatively, the lining or the packing may be formed of a metal having a high electrode potential.

During the distillation, the olefins represented by the formula (3) and the alcohols represented by the formula (4) are withdrawn from the top of the distillation column and then the desired mixture of fluorine-containing (meth)acrylic esters is withdrawn from the side of the distillation column or as the still residue or the bottom. The most of the olefins and the alcohols distilled off is from C₂F₅(CF₂CF₂)ₙI with n=3 and n=4 and the boiling points of these olefins and alcohols are low. Therefore, the mixture of the fluorine-containing (meth)acrylic esters with a high purity can be obtained even if the bottom temperature falls in the low range as described above.

Contrariwise, when the fluoroalkyl iodides of n≥5 is not sufficiently reduced in the step (A), the fluorine-containing alcohol of n=5 is contained in the mixture to be subjected to the distillation step in the step (D). In that case, there is a disadvantage that the theoretical plate number needs to be a large one in order to remove the fluorine-containing alcohol of n=5 since the boiling point of this fluorine-containing alcohol is particularly close to those of the fluorine-containing (meth)acrylic esters of n=3 and 4. Further, the bottom temperature is required to be high in order to withdraw, as the object, the fluorine-containing (meth)acrylic esters of n≥5, together with the esters of n=3 and 4 because the esters of n≥5 have high boiling points. When the bottom temperature is high, the polymerization is promoted and therefore the inhibitor of polymerization needs to be added in a larger amount. The larger amount of the polymerization inhibitor added causes a disadvantage that the impurities derived from the inhibitor are increased. For this reason, the use of the fluoroalkyl iodides of n≥5 is not desirable from the viewpoints of the efficiency of the distillation step after the step (C) and the quality of the intended mixture obtained after the distillation step. The step (D) is preferably conducted as the distillation step, but this step is not limited to the distillation.

A fluorine-containing (meth)acrylate-based polymer can be produced by subjecting the mixture obtained in the step (D) to the polymerization step. The polymerization may be carried out employing any polymerization conditions which are conventionally employed. The resultant polymer has excellent quality since it contains less impurities. The resultant polymer is useful as the water- and oil-repellent for treating a surface of a substrate, such as textile products, stone material, a filter (for example, an electrostatic filter), a dust-protective mask, a fuel battery, glass, paper, wood, leather, fur skin, asbestos, brick, cement, metal and oxide, ceramic material, and plastics. Further, the resultant polymer is useful as a water- and oil-repellent and antifouling finish for carpeting.

In the second production method, a step of obtaining a mixture of the fluoroalkyl iodides of n≥0 in which step the mixture of the fluoroalkyl iodides obtained by the telomerization reaction is not subjected to distillation, is carried out as the step (A') instead of the step (A) of the first production method. Next, the step of adding ethylene to the mixture of the fluoroalkyl iodides obtained in the step (A') is carried out (the step (B)) and then a step of obtaining another mixture of the ethylene adducts which contains the ethylene adducts of n=3 and n=4 in an amount of 85 mol% or more, by subjecting the resultant mixture to the distillation (the step (B')). The specific method of ethylene addition in the step (B) of the second production method is as described in conjunction with the step (B) of the first production method, and therefore the details thereof are omitted herein.

When the step (B') is carried out as the distillation step, the distillation is preferably carried out so that the mixing proportion of the sum of the ethylene adducts of n≤2 and n≥5 is less than 15 mol% and more preferably carried out so that the mixing proportion of the sum of the ethylene adducts of n≤2 is less than 10 mol% and the mixing proportion of the ethylene adducts of n≥5 is less than 5 mol%. The reason therefor is, as described in conjunction with the step (A) of the first production method, is to prevent the ethylene adducts with n≥5 which are difficult to be separated from the ester mixture from being fed to the step (C). The specific conditions for distillation (generally, rectification) are the bottom temperature of from 60°C to 140°C, the pressure in the column of from 0.5kPa to 2kPa and the theoretical plate number of from 10 to 25.

The steps (C) and (D) in the second production method are carried out in the same manner as those in the first production method and the resultant mixture is as the same as that obtained in the first production method. Therefore, the details of those are omitted herein.

### Examples

### (Example 1)

A mixture of fluoroalkyl alcohols represented by C₂F₅(CF₂CF₂)ₙI was produced as follows. 100g of CF₃CF₂I as a telogen was charged into a reactor together with 10g of a copper catalyst. These are stirred to give a slurry in which the copper catalyst was suspended and the slurry was heated to 80°C. Tetrafluoroethylene as a taxogen was charged into the reactor with the temperature inside the reactor kept at 80°C and the reaction pressure was maintained at 0.8MPa. At the time when 10g of tetrafluoroethylene was charged, it was confirmed by gas chromatography that the telomerization reaction proceeded.

The resultant mixture of the fluoroalkyl iodides was subjected to distillation selecting the theoretical number of ten(10), the bottom temperature of from 60°C to 140°C and the pressure in the column of from 100 to 10kPa. The telomers with smaller "n" values were withdrawn from the column top sequentially and then a mixture containing the fluoroalkyl iodides of n=3 and n=4 in an amount of 91 mol% in total was drawn from the column top and this mixture was used in the next ethylene addition step. More specifically, this mixture contained the fluoroalkyl iodides with n≤2 in an amount of 5 mol%, one with n=3 in an amount of 76 mol%, one with n=4 in an amount of 17 mol% and one with n≥5 in an amount of 2 mol%.

The mixture obtained in this manner was subjected to ethylene addition to give ethylene adducts. The ethylene addition step was carried out as follows. 100g of the fluoroalkyl iodide mixture and 5g of copper catalyst as a catalyst were charged into an autoclave and then heated to 100°C. Next, an ethylene gas was charged into a gas phase so that the reaction pressure was kept at 0.3MPa, and the reaction was made for three hours. As a result, a mixture of the ethylene adducts was obtained. An yield of the ethylene adducts was 99 mass%.

Next, an esterification step was carried out according to the following procedures. 1576g (2.67 mol) of the mixture of the ethylene adducts obtained by carrying out the ethylene addition step according to the above procedures, 320g (2.90 mol) of potassium acrylate, 680mL of tert-butyl alcohol, and 1.8g of hydroquinone and 0.32g of hydroquinone monomethylether were firstly charged into an autoclave with a volume of 3L and heated to 180°C-190°C and reacted for 6 hours. After the reaction, a reaction mixture was cooled. Next, KI as a byproduct was removed by filtration. Thereafter, a filtrate was subjected to distillation to remove tert-butyl alcohol and then a reaction mixture having a composition shown in Table 1 was obtained. 1000g of this reaction mixture was measured and charged into a still and then subjected to distillation using a rectifier with theoretical plate number of ten(10) and selecting the pressure inside the rectifier of 0.9kPa and the still temperature of 160°C. As a result, a mixture after distillation having a composition as shown in Table 1 was obtained as a distillate liquid In Table 1, the compositions of the reaction mixture and the mixture after distillation were determined by gas chromatography.

**Table 1**

| Component | Reaction mixture (mol%) | Mixture after distillation (mol%) |
|---|---|---|
| C₆F₁₃CH=CH₂ | 0.91 | ND |
| C₈F₁₇CH=CH₂ | 13.65 | ND |
| C₁₀F₂₁CH=CH₂ | 3.09 | ND |
| C₁₂F₂₅CH=CH₂ | 0.34 | 0.01 |
| C₆F₁₃CH₂CH₂OH | 0.11 | ND |
| C₈F₁₇CH₂CH₂OH | 1.57 | ND |
| C₁₀F₂₁CH₂CH₂OH | 0.35 | 0.04 |
| C₁₂F₂₅CH₂CH₂OH | 0.04 | 0.05 |
| C₆F₁₃CH₂CH₂OCOCH=CH₂ | 4.09 | 5.1 |
| C₈F₁₇CH₂CH₂OCOCH=CH₂ | 60.73 | 75.93 |
| C₁₀F₂₁CH₂CH₂OCOCH=CH₂ | 13.54 | 16.92 |
| C₁₂F₂₅CH₂CH₂OCOCH=CH₂ | 1.58 | 1.96 |

As shown in Table 1, the impurities contained in the reaction mixture, specifically, C₈F₁₇CH=CH₂, C₁₀F₂₁CH=CH₂, C₈F₁₇CH₂CH₂OH and C₁₀F₂₁CH₂CH₂OH were able to be removed considerably by the distillation. As a result, the highly-pure mixture of the fluorine-containing acrylic esters was obtained, in which the contents of the fluorine-containing acrylic esters of n=3 and n=4 contained were large. Specifically, the mixture contained, as the impurities, C₁₀F₂₁CH₂CH₂OH (n=4) in an amount of 0.04 mol%, C₁₂F₂₅CH=CH₂ (n=5) in an amount of 0.01 mol% and C₁₂F₂₅CH₂CH₂OH (n=5) in an amount of 0.05 mol%.

### (Example 2)

The reaction mixture having a composition shown in Table 2 was obtained in the same manner as that in Example 1 except that the rectification was carried out so that more initial distillate and more final distillate were cut and the fluoroalkyl iodides of n=2 and n=5 were not contained in the mixture, to give a mixture which contains the fluoroalkyl iodide of n=3 in an amount of 80 mol% and the fluoroalkyl iodide of n=4 in an amount of 20 mol%. 1000g of this reaction mixture was measured, charged into a still and subjected to continuous distillation using a rectifier with the theoretical plate number of ten(10) and selecting the pressure in the rectifier of 0.9kPa and the still temperature of 160°C. As a result, a mixture after distillation having a composition as shown in Table 2 was obtained as a distillate liquid In Table 2, the compositions of the reaction mixture and the mixture after distillation were determined by gas chromatography.

**Table 2**

| Component | Reaction mixture (mol%) | Mixture after distillation (mol%) |
|---|---|---|
| C₈F₁₇CH=CH₂ | 13.54 | ND |
| C₁₀F₂₁CH=CH₂ | 3.41 | ND |
| C₈F₁₇CH₂CH₂OH | 0.88 | ND |
| C₁₀F₂₁CH₂CH₂OH | 0.18 | 0.03 |
| C₈F₁₇CH₂CH₂OCOCH=CH₂ | 65.58 | 80.31 |
| C₁₀F₂₁CH₂CH₂OCOCH=CH₂ | 16.41 | 19.66 |

As shown in Table 2, the highly-pure mixture of the fluorine-containing acrylic esters with less impurities was obtained also in Example 2 similarly to Example 1. Specifically, only C₁₀F₂₁CH₂CH₂OH (n=4) was contained in an amount of 0.03 mol% as the impurity.

### (Comparative Example 1)

The reaction mixture having a composition as shown in Table 3 was obtained in the same manner as that in Example 1 except that the rectification conditions of the fluoroalkyl iodide mixture were changed and the rectification after the telomerization reaction was carried out so that a mixture wherein only the fluoroalkyl iodides of n≤2 were removed (that is, a mixture of the fluoroalkyl iodides of n≥3) was obtained by carrying out the rectification, and then the mixture was used in the ethylene addition step. 1000g of this reaction mixture was measured, charged into a still and subjected to distillation using a rectifier with the theoretical plate number of ten(10) and selecting the pressure inside the rectifier of 0.9kPa and the still temperature of 160°C. As a result, a mixture after distillation having a composition as shown in Table 3 was obtained as a still residue. In Table 3, the compositions of the reaction mixture and the mixture after distillation were determined by gas chromatography.

**Table 3**

| Component | Reaction mixture (mol%) | Mixture after distillation (mol%) |
|---|---|---|
| C₈F₁₇CH=CH₂ | 5.40 | ND |
| C₁₀F₂₁CH=CH₂ | 3.46 | ND |
| C₁₂F₂₅CH=CH₂ | 1.53 | 0.01 |
| C₁₄F₂₉CH=CH₂ | 0.21 | 0.26 |
| C₁₆F₃₃CH=CH₂ | 0.11 | 0.12 |
| C₈F₁₇CH₂CH₂OH | 1.28 | ND |
| C₁₀F₂₁CH₂CH₂OH | 0.69 | 0.04 |
| C₁₂F₂₅CH₂CH₂OH | 0.32 | 0.37 |
| C₁₄F₂₉CH₂CH₂OH | 0.12 | 0.15 |
| C₆F₁₃CH₂CH₂OCOCH=CH₂ | 0.36 | ND |
| C₈F₁₇CH₂CH₂OCOCH=CH₂ | 52.80 | 61.06 |
| C₁₀F₂₁CH₂CH₂OCOCH=CH₂ | 20.91 | 24.16 |
| C₁₂F₂₅CH₂CH₂OCOCH=CH₂ | 7.60 | 8.80 |
| C₁₄F₂₉CH₂CH₂OCOCH=CH₂ | 2.28 | 2.66 |
| C₁₆F₃₃CH₂CH₂OCOCH=CH₂ | 1.40 | 1.64 |
| C₁₈F₃₇CH₂CH₂OCOCH=CH₂ | 0.24 | 0.30 |

The impurities derived from CₙF₂ₙI of n≥5 is contained in addition to the impurities derived from CₙF₂ₙI of n=3 and n=4 in the mixture of the fluorine-containing acrylic esters obtained in this comparative example. Of these impurities, the alcohols and the olefins with smaller "n" values were able to be removed considerably, but the alcohols and the olefins with larger "n" values remained in the mixture after distillation. Specifically, the proportion of the sum of the impurities (the alcohols and the olefins) was 0.95 mol% and the proportion of C₁₀F₂₁CH₂CH₂OH (n=5) was 0.37 mol%.

As described above, the mixture of the fluorine-containing (meth)acrylic esters can be obtained wherein the proportion of the impurities is reduced to a ppm level according to the production method of the present invention. The monomer mixture with less impurities is useful for producing a polymer of high quality. Further, the method of the present invention makes it possible to give the mixture which contains the fluorine-containing (meth) acrylic esters with "n" values of 3 and 4 at a high ratio and the fluorine-containing (meth)acrylic esters with n≤2 or n≥5 at a low ratio. As described above, the fluorine-containing (meth)acrylic esters with n=3 and 4 are monomers which produce the polymer useful as the water- and oil-repellent, and therefore the production method of the present invention contributes to improvement in the quality of the polymer in this point.

### Industrial Applicability

The production method of the present invention makes it possible to obtain the mixture of the fluorine-containing (meth)acrylic esters represented by the formula C₂F₅(CF₂CF₂)ₙCH₂CH₂OCOCR¹=CH₂ which mixture contains a high proportion of the esters of n=3 and n=4 and a low proportion of the other compounds. Therefore, the mixture of the fluorine-containing (meth)acrylic esters obtained by this production method is suitable for being used as the monomer for producing the polymer which is particularly useful as the water- and oil-repellent.

## Claims

1. A method for producing a mixture of fluorine-containing (meth)acrylic esters each of which esters is represented by the formula (1) :
C₂F₅(CF₂CF₂)ₙCH₂CH₂OCOCR¹=CH₂ (1)
wherein R¹ is a hydrogen atom or a methyl group and "n" is an integer of at least zero, which comprises:
(A) a step of obtaining a mixture of fluoroalkyl iodides each of which is represented by C₂F₅(CF₂CF₂)ₙI
wherein "n" is an integer of at least zero which mixture contains the fluoroalkyl iodides of n=3 and n=4 in an amount of 85 mol% or more in total;
(B) a step of ethylene addition wherein ethylene is added to the mixture of the fluoroalkyl iodides obtained in the step (A) to give a mixture of ethylene adducts each of which is represented by the formula (2):
C₂F₅(CF₂CF₂)ₙCH₂CH₂I (2)
wherein "n" is an integer of at least zero;
(C) an esterification step of reacting the mixture of the ethylene adducts obtained in the step (B) with a (meth)acrylic compound to give a mixture containing the fluorine-containing (meth)acrylic esters; and
(D) a step of reducing a proportion of fluorine-containing olefins represented by the formula (3) and a proportion of fluorine-containing alcohols represented by the formula (4) contained in the mixture obtained in the step (C) :
C₂F₅(CF₂CF₂)ₙCH=CH₂ (3)
wherein "n" is an integer of at least zero,
C₂F₅(CF₂CF₂)ₙCH₂CH₂OH (4)
wherein "n" is an integer of at least zero.

2. The method according to claim 1 wherein a proportion of the fluoroalkyl iodides represented by the formula (1) with "n" of 0, 1 and 2 is less than 10 mol% and a proportion of the fluoroalkyl iodides with "n" of 5 or more is less than 5 mol% in the mixture obtained in the step (A).

3. A method for producing a mixture of fluorine-containing (meth)acrylic esters each of which esters is represented by the formula (1) :
C₂F₅(CF₂CF₂)ₙCH₂CH₂OCOCR¹=CH₂ (1)
wherein R¹ is a hydrogen atom or a methyl group and "n" is an integer of at least zero, which comprises:
(A') a step of obtaining a mixture of fluoroalkyl iodides each of which is represented by C₂F₅(CF₂CF₂)ₙI
wherein "n" is an integer of at least zero;
(B) a step of ethylene addition wherein ethylene is added to the mixture of the fluoroalkyl iodides obtained in the step (A') to give a mixture of ethylene adducts each of which is represented by the formula (2):
C₂F₅(CF₂CF₂)ₙCH₂CH₂I (2)
wherein "n" is an integer of at least zero;
(B') a step of obtaining, from the mixture of the ethylene adducts obtained in the step (B), another mixture of ethylene adducts which contains the adducts of n=3 and n=4 in an amount of 85 mol% or more in total;
(C) an esterification step of reacting the mixture of the ethylene adducts obtained in the step (B') with a (meth)acrylic compound to give a mixture containing the fluorine-containing (meth)acrylic esters; and
(D) a step of reducing a proportion of fluorine-containing olefins represented by the formula (3) and a proportion of fluorine-containing alcohols represented by the formula (4) contained in the mixture obtained in the step (C):
C₂F₅(CF₂CF₂)ₙCH=CH₂ (3)
wherein "n" is an integer of at least zero,
C₂F₅(CF₂CF₂)ₙCH₂CH₂OH (4)
wherein "n" is an integer of at least zero.

4. The method according to claim 3 wherein a proportion of the ethylene adducts represented by the formula (2) with "n" of 0, 1 and 2 is less than 10 mol% and a proportion of the ethylene adducts with "n" of 5 or more is less than 5 mol% in the mixture obtained in the step (B').

5. The method according to claim 1, wherein the step (D) is carried out by subjecting the mixture obtained in the step (C) to distillation.

6. The method according to claim 3, wherein the step (D) is carried out by subjecting the mixture obtained in the step (C) to distillation.

7. The method according to claim 5 wherein the distillation is conducted in the step (D) so that a proportion of the fluorine-containing olefins represented by the formula (3) with "n" of 0, 1 and 2 is substantially 0 mol%, a proportion of the fluorine-containing olefin with "n" of 3 is substantially 0 mol%, a proportion of fluorine-containing olefin with "n" of 4 is substantially 0 mol%, and a proportion of the fluorine-containing olefins with "n" of 5 or more is from 0 mol% to 0.1 mol%, based on the total moles of the fluorine-containing compounds obtained as the distillate.

8. The method according to claim 6 wherein the distillation is conducted in the step (D) so that a proportion of the fluorine-containing olefins represented by the formula (3) with "n" of 0, 1 and 2 is substantially 0 mol%, a proportion of the fluorine-containing olefin with "n" of 3 is substantially 0 mol%, a proportion of fluorine-containing olefin with "n" of 4 is substantially 0 mol%, and a proportion of the fluorine-containing olefins with "n" of 5 or more is from 0 mol% to 0.1 mol%, based on the total moles of the fluorine-containing compounds obtained as the distillate.

9. The method according to claim 5 wherein the distillation is conducted in the step (D) so that the distillation is conducted so that a proportion of the fluorine-containing alcohols represented by the formula (4) with "n" of 0, 1 and 2 is substantially 0 mol%, a proportion of the fluorine-containing alcohol with "n" of 3 is substantially 0 mol%, a proportion of fluorine-containing alcohol with "n" of 4 is from 0 mol% to 0.05 mol%, and a proportion of the fluorine-containing alcohols with "n" of 5 or more is from 0 mol% to 0.1 mol%, based on the total moles of the fluorine-containing compounds obtained as the distillate.

10. The method according to claim 6 wherein the distillation is conducted in the step (D) so that the distillation is conducted so that a proportion of the fluorine-containing alcohols represented by the formula (4) with "n" of 0, 1 and 2 is substantially 0 mol%, a proportion of the fluorine-containing alcohol with "n" of 3 is substantially 0 mol%, a proportion of fluorine-containing alcohol with "n" of 4 is from 0 mol% to 0.05 mol%, and a proportion of the fluorine-containing alcohols with "n" of 5 or more is from 0 mol% to 0.1 mol%, based on the total moles of the fluorine-containing compounds obtained as the distillate.

11. A method for producing a polymer of fluorine-containing (meth)acrylic esters each of which is represented by a formula (1) :
C₂F₅(CF₂CF₂)ₙCH₂CH₂OCOCR¹=CH₂ (1)
wherein R¹ is a hydrogen atom or a methyl group and "n" is an integer of at least zero, which comprises:
(A) a step of obtaining a mixture of fluoroalkyl iodides each of which is represented by C₂F₅(CF₂CF₂)ₙI
wherein "n" is an integer of at least zero which mixture contains the fluoroalkyl iodides of n=3 and n=4 in an amount of 85 mol% or more in total;
(B) a step of ethylene addition wherein ethylene is added to the mixture of the fluoroalkyl iodides obtained in the step (A) to give a mixture of ethylene adducts each of which is represented by the formula (2):
C₂F₅(CF₂CF₂)ₙCH₂CH₂I (2)
wherein "n" is an integer of at least zero;
(C) an esterification step of reacting the mixture of the ethylene adducts obtained in the step (B) with a (meth)acrylic compound to give a mixture containing the fluorine-containing (meth)acrylic esters;
(D) a step of reducing a proportion of fluorine-containing olefins represented by the formula (3) and a proportion of fluorine-containing alcohols represented by the formula (4) contained in the mixture obtained in the step (C) :
C₂F₅(CF₂CF₂)ₙCH=CH₂ (3)
wherein "n" is an integer of at least zero,
C₂F₅(CF₂CF₂)ₙCH₂CH₂OH (4)
wherein "n" is an integer of at least zero; and
(E) a step of polymerizing a mixture obtained in the step (D).

12. A method for producing a polymer of fluorine-containing (meth)acrylic esters each of which is represented by a formula (1):
C₂F₅(CF₂CF₂)ₙCH₂CH₂OCOCR¹=CH₂ (1)
wherein R¹ is a hydrogen atom or a methyl group and "n" is an integer of at least zero, which comprises:
(A') a step of obtaining a mixture of fluoroalkyl iodides each of which is represented by C₂F₅(CF₂CF₂)ₙI
wherein "n" is an integer of at least zero;
(B) a step of ethylene addition wherein ethylene is added to the mixture of the fluoroalkyl iodides obtained in the step (A') to give a mixture of ethylene adducts each of which is represented by the formula (2):
C₂F₅(CF₂CF₂)ₙCH₂CH₂I (2)
wherein "n" is an integer of at least zero;
(B') a step of obtaining, from the mixture of the ethylene adducts obtained in the step (B), another mixture of ethylene adducts which contains the adducts of n=3 and n=4 in an amount of 85 mol% or more in total;
(C) an esterification step of reacting the mixture of the ethylene adducts obtained in the step (B') with a (meth)acrylic compound to give a mixture containing the fluorine-containing (meth)acrylic esters; and
(D) a step of reducing a proportion of fluorine-containing olefins represented by the formula (3) and a proportion of fluorine-containing alcohols represented by the formula (4) contained in the mixture obtained in the step (C):
C₂F₅(CF₂CF₂)ₙCH=CH₂ (3)
wherein "n" is an integer of at least zero,
C₂F₅(CF₂CF₂)ₙCH₂CH₂OH (4)
wherein "n" is an integer of at least zero; and
(E) a step of polymerizing a mixture obtained in the step (D).

13. A fluorine-containing (meth)acrylate polymer produced by the method according to claim 11.

14. A fluorine-containing (meth)acrylate polymer produced by the method according to claim 12.
